# EUROPÄISCHE PATENTSCHRIFT

(11) **EP 1 209 236 B1**
(45) Veröffentlichungstag und Bekanntmachung des Hinweises auf die Patenterteilung: **15.09.2004**
(21) Anmeldenummer: 01126453.8
(22) Anmeldetag: 09.11.2001
(51) Int. Cl.: C12N 15/82, C12N 15/54, C12N 9/12, C12N 5/10, C07K 16/40, C12Q 1/68, G01N 33/50, G01N 33/68, A01N 25/00

(54) **Phosphomevalonat Kinasen aus Pflanzen**
phosphomevalonate kinases from plants
phosphomevalonate kineses d'origine vegetale

(30) Priorität: 22.11.2000 DE 10057755
(43) Veröffentlichungstag der Anmeldung: 29.05.2002
(73) Patentinhaber: Bayer CropScience AG, 40789 Monheim (DE)
(72) Erfinder: Meissner, Ruth, Dr., 51381 Leverkusen (DE); Lechelt-Kunze, Christa, Dr., 50935 Köln (DE)

(56) Entgegenhaltungen:
- WO-A-00/15809
- WO-A-00/53782
- WO-A-01/14533
- DATABASE EMBL [Online] Accession number AAB18130, 8. November 2000 (2000-11-08) XP002191330
- DATABASE EMBL [Online] Accession number AA660847, 14. November 1997 (1997-11-14) XP002191331
- DATABASE EMBL [Online] Accession number BF070746, 18. Oktober 2000 (2000-10-18) XP002191332
- DATABASE EMBL [Online] Accession number AC079041, 18. August 2000 (2000-08-18) XP002191333 -& DATABASE EMBL [Online] Accession number Q9C6T1 , 1. Juni 2001 (2001-06-01) XP002191334
- DATABASE EMBL [Online] Accession number AF429385, 27. Oktober 2001 (2001-10-27) XP002191335
- SCHULTE A E ET AL: "Purification and characterization of phosphomevalonate kinase from Catharanthus roseus" PHYTOCHEMISTRY, PERGAMON PRESS, GB, Bd. 52, Nr. 6, November 1999 (1999-11), Seiten 975-983, XP004291102 ISSN: 0031-9422
- TSAY Y H ET AL: "CLONING AND CHARACTERIZATION OF ERG8, AN ESSENTIAL GENE OF SACCHAROMYCES CEREVISIAE THAT ENCODES PHOSPHOMEVALONATE KINASE" MOLECULAR AND CELLULAR BIOLOGY, AMERICAN SOCIETY FOR MICROBIOLOGY, WASHINGTON, US, Bd. 11, Nr. 2, Februar 1991 (1991-02), Seiten 620-631, XP000981936 ISSN: 0270-7306
- HOUTEN SANDER M ET AL: "Nonorthologous gene displacement of phosphomevalonate kinase." MOLECULAR GENETICS AND METABOLISM, Bd. 72, Nr. 3, März 2001 (2001-03), Seiten 273-276, XP001038655 ISSN: 1096-7192
- BACH T J ET AL: "CLONING OF CDNAS OR GENES ENCODING ENZYMES OF STEROL BIOSYNTHESIS FROM PLANTS AND OTHER EUKARYOTES: HETEROLOGOUS EXPRESSION AND COMPLEMENTATION ANALYSIS OF MUTATIONS FOR FUNCTIONAL CHARACTERIZATION" PROGRESS IN LIPID RESEARCH, PERGAMON PRESS, PARIS, FR, Bd. 36, Nr. 2/3, September 1997 (1997-09), Seiten 197-226, XP000957903 ISSN: 0163-7827
- DATABASE EMBL [Online] Accession number Q9UT88, 1. Mai 2000 (2000-05-01) XP002191336
- NORMAN M A ET AL: "SITE OF CLOMAZONE ACTION IN TOLERANT SOYBEAN AND SUSCEPTIBLE COTTON PHOTOMIXOTROPHIC CELL SUSPENSION CULTURES" PLANT PHYSIOLOGY (BETHESDA), Bd. 94, Nr. 2, 1990, Seiten 704-709, XP001042396 ISSN: 0032-0889

## Beschreibung

Die Erfindung betrifft Nukleinsäuren, die für pflanzliche Polypeptide mit der biologischen Aktivität von Phosphomevalonat Kinasen kodieren, die davon die kodierten Polypeptide sowie deren Verwendung als Targets für Herbizide und deren Verwendung zum Identifizieren von neuen, herbizid wirksamen Verbindungen sowie Verfahren zum Auffinden von Modulatoren dieser Polypeptide.

Unerwünschtes Pflanzenwachstum kann durch die Verwendung von Herbiziden verhindert werden. Die Ansprüche an Herbizide sind dabei hinsichtlich ihrer Wirksamkeit, Kosten und Umweltverträglichkeit stetig angestiegen. Es existiert deshalb ein Bedarf an neuen Substanzen, die zu leistungsfähigen neuen Herbiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt.

Vorteilhafte Angriffspunkte für Herbizide werden in essentiellen Biosynthesewegen gesucht. So führt die Biosynthese von Isoprenoiden in Pflanzen u.a. zur Synthese von Carotinoiden sowie der Seitenketten des Plastochinons und des Chlorophylls. Diese Produkte sind für das photosynthetische Wachstum von Pflanzen unerläßlich. Die Inhibition eines Schrittes in diesem Biosyntheseweg führt zur Beendigung des Wachstums einer Pflanze. Des weiteren werden aus Isoprenoiden Pflanzenhormone wie Gibberellinsäure, Abscisinsäure und Brassinosteroide und Membrankomponenten (Phytosterole) gebildet, die auch für das Wachstum der Pflanze essentiell sind.

Isopentyldiphosphat (IPP) ist der Verzweigungspunkt von dem aus die verschiedensten Isoprenoide gebildet werden. Die Herstellung von IPP ist daher ein kritischer Punkt im Pflanzenstoffwechsel. In Pflanzen wird IPP über zwei verschiedene Stoffwechselwege in verschiedenen Kompartimenten hergestellt. Im Endoplasmatischen Retikulum (ER) und im Cytosol verläuft die Synthese von IPP über den klassischen Acetat/Mevalonat-Stoffwechselweg, wie er auch im tierischen Organismus abläuft. Dagegen wird in Chloroplasten IPP über den alternativen Glycerinaldehydphosphat/Pyruvat-Stoffwechselweg synthetisiert. Beide Stoffwechselwege sind essentiell, da verschiedene isoprenoide Metaboliten in den verschiedenen Kompartimenten gebildet werden. Ausserdem ist noch nicht geklärt inwieweit die beiden Stoffwechselwege autonom sind oder Metabolitenaustausch zwischen den Kompartimenten stattfindet (Heintze et al., 1990, Kleinig, 1989).

Clomazone ist eine bekannte herbizide Verbindung, die den Gehalt an Carotinoiden und Chlorophyll im Blatt verringert. Es wurde lange Zeit angenommen, dass Clomazone über die Hemmung des Isoprenoid-Stoffwechselwegs wirkt. Norman et al. (1990) hatten gezeigt, dass der Wirkort zwischen Mevalonat und Geranylgeranyl Pyrophosphat liegen müsste. Das würde den Wirkort auf eines der dazwischen liegenden fünf Enzyme, von denen eines die Phosphomevalonat Kinase ist, festlegen. Etwas neuere Arbeiten von Weimer et al. (1992) und Rodney Croteau (1992) weisen allerdings darauf hin, dass der Angriffspunkt von Clomazone an einer anderen Stelle zu suchen ist.

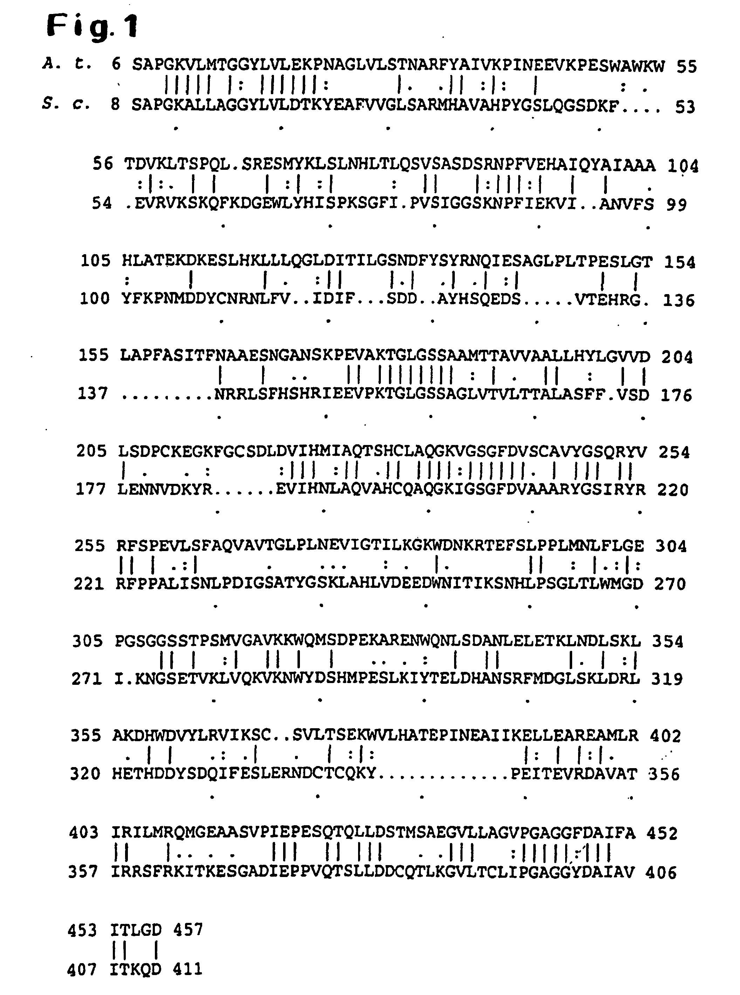

Im Rahmen der vorliegenden Erfindung wurde eine cDNA aus *Arabidopsis thaliana* cv. Columbia mit Homologie zur Phophomevalonat Kinase, im folgenden mit PMVK abgekürzt, aus *Saccharomyces cerevisiae* isoliert (Abb. 1). Dieses Gen konnte in *Arabidopsis thaliana cv.* Columbia durch Behandlung mit dem Herbizid Chlorsulfuron (10g/ha) induziert werden.

Die Homologie zwischen der *Saccharomyces cerevisiae* PMVK (= ERG8) und der aus *A. thaliana* isolierten cDNA beträgt 44% Ähnlichkeit bzw. 35% Identität (siehe Abb. 1, Bestfit mit Wisconsin Package Version 10.1). Dies entspricht z.B. der Homologie zwischen der *Saccharomyces cerevisiae* Mevalonat Kinase und der *Arabidopsis thaliana* Mevalonat Kinase mit einer Ähnlichkeit von 45 % und einer Identität von 35 %. Für die Mevalonat Kinase aus *Arabidopsis thaliana* konnte die Funktion durch Komplementation der entsprechenden Mutante aus *Saccharomyces cerevisiae* nachgewiesen werden. Des weiteren weist die im Rahmen der vorliegenden Erfindung isolierte cDNA eine 69 %ige Identität zu einer partiellen PMVK-Sequenz aus *Pinus radiata* gemäß SEQ ID NO:5 auf, die zur Modifikation von Isoprenoid-Gehalt, Isoprenoid- Zusammensetzung und Isoprenoid-Stoffwechsel von Pflanzen von Interesse ist (WO 00/36 081). Weitere partielle cDNAs aus Pflanzen (*Medicago trunculata*, Accession Number AA660847, siehe SEQ ID NO:3 und *Gossypium hirsutum*, Accession Number AI727861, siehe SEQ ID NO:4) sind als putative PMVKs isoliert worden. In Datenbanken sind aus verschiedenen Sequenzierungsprojekten verschiedene Sequenzen (ESTs und genomische Sequenzen) aus Arabidopsis spp. zu finden, die der hier isolierten PMVK-Sequenz oder Teilen davon entsprechen, allerdings werden zu diesen Sequenzen oder Sequenzfragmenten keine Angaben zu Funktion oder Bedeutung gemacht.

Auch die Phosphomevalonatkinase aus *Catharanthus roseus* wurde bereits beschrieben (Schulte et al. (1999): "Purification and characterization of phosphomevalonate kinase from *Catharanthus roseus* "Phytochemistry 52 (6), 975-983) und die Abhängigkeit der Enzymaktivität der Phosphomevalonatkinase von Kationen untersucht.

Durch die vorliegende Erfindung wird nun die vollständige cDNA Sequenz einer pflanzlichen Phosphomevalonat Kinase zur Verfügung gestellt und deren Verwendung bzw. die Verwendung des davon kodierten Polypeptids zur Identifizierung neuer herbizider Wirkstoffe beschrieben.

Im Rahmen der vorliegenden Erfindung werden deshalb Nukleinsäuren beschrieben, die für vollständige pflanzliche Phosphomevalonat Kinasen kodieren, ebenso die bekannten partiellen Nukleinsäuresequenzen aus *Medicago trunculata* gemäß SEQ ID NO:3, *Gossypium hirsutum* gemäß SEQ ID NO:4 und aus *Pinus radiata* gemäß SEQ ID NO:5.

Im Rahmen der vorliegenden Erfindung werden so Nukleinsäuren beschrieben, die für die Phosphomevalonat Kinase aus *Arabidopsis thaliana* kodieren und unter SEQ ID NO:1 beschrieben sind und/oder für ein Polypeptid gemäß SEQ ID NO:2 oder aktive Fragmente davon kodieren.

Bei diesen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt handelt es sich bei diesen Nukleinsäuren um DNA-Fragmente, die der cDNA von Arabidopsispflanzen entsprechen.

Besonders bevorzugt umfassen die im Rahmen der vorliegenden Erfindung beschriebenen Nukleinsäuren eine Sequenz ausgewählt aus
a) der Sequenz gemäß SEQ ID NO: 1,
b) Sequenzen, die für ein Polypeptid codieren, welches die Aminosäuresequenz gemäß SEQ ID NO: 2 umfasst,
c) zumindest 14 Basenpaare langen Teilsequenzen der unter a) oder b) definierten Sequenzen,
d) Sequenzen, welche an die unter a) oder b) definierten Sequenzen bei einer Hybridisierungstemperatur von 35-52°C hybridisieren,
e) Sequenzen, welche eine zumindest 70 %ige, bevorzugt eine 85 %ige, besonders bevorzugt eine 90 %ige Identität, ganz besonders bevorzugt eine 95 %ige Identität mit den unter a) definierten Sequenzen aufweisen,
f) Sequenzen, welche eine zumindest 70 %ige, bevorzugt eine 80 %ige, besonders bevorzugt eine 90 %ige Identität, ganz besonders bevorzugt eine 95 %ige Identität mit den unter b) definierten Sequenzen aufweisen,
g) Sequenzen, welche zu den unter a) oder b) definierten Sequenzen komplementär sind, und
h) Sequenzen, welche aufgrund der Degeneriertheit des genetischen Codes für dieselbe Aminosäuresequenz codieren wie die unter a) bis f) definierten Sequenzen.

Besonderes Augenmerk liegt dabei auf der Nukleinsäure , die ein cDNA-Molekül mit der Sequenz gemäß SEQ ID NO: 1 darstellt.

Der Ausdruck "vollständige" Phosphomevalonat Kinase wie er hierin verwendet wird, beschreibt die Phosphomevalonat-Kinase, die kodiert wird von einer vollständigen kodierenden Region einer Transkriptionseinheit beginnend mit dem ATG-Startcodon und umfassend alle informationstragenden Exonbereiche des im Herkunftsorganismus vorliegenden, für die Phosphomevalonat-Kinase kodierenden Gens, sowie die für eine korrekte Termination der Transkription nötigen Signale.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Pflanzen als Arabidopsis isoliert werden, welche für Phosphomevalonat Kinasen kodieren, welche dieselben oder ähnliche Eigenschaften wie die Kinase mit der Aminosäuresequenz gemäß SEQ ID NO: 2 aufweisen.

Der Ausdruck "cDNA" wie er hierin verwendet wird, ist die Bezeichnung für die einzel- bzw. doppelsträngige Kopie eines RNA-Moleküls und ist deshalb als Kopie biologisch aktiver mRNA intronfrei, d.h. alle kodierenden Regionen eines Gens sind in zusammenhängender Form enthalten.

Hybridisierungsbedingungen werden nach folgender Formel näherungsweise berechnet:

Schmelztemperatur Tm = 81.5 °C + 16.6 log[c(Na⁺)] + 0.41(%G + C)) - 500/n (Lottspeich und Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na⁺ (entspricht 0.1 x SSC) gewaschen. Wird eine RNA-Sonde zur Hybridisierung verwendet, so ist der Schmelzpunkt um 10-15 °C höher.

Bevorzugte Hybridisierungsbedingungen sind nachstehend angegeben:
Hybridisierungslösung: DIG Easy Hyb (Fa.: Roche)
Hybridisierungstemperatur: 35-52°C, bevorzugt 42°C (DNA-DNA) bzw. 50°C (DNA-RNA).
   1. Waschschritt: 2x SSC, 2 mal 5 min bei Raumtemperatur;
   2. Waschschritt: 2 mal 15min in 1x SSC, bei 50°C; bevorzugt 0,5x SSC, bei 65°C; besonders bevorzugt 0,2x SSC, bei 65°C.

Der Grad der Identität der Nukleinsäuren wird vorzugsweise bestimmt mit Hilfe des Programms NCBI BLASTN Version 2.0.14. (Altschul et al., 1997).

Im Rahmen der vorliegenden Erfindung werden weiterhin DNA-Konstrukte beschrieben, die eine der vorstehend genannten Nukleinsäuren und einen homologen oder heterologen Promotor umfassen.

Der Ausdruck "homologer Promotor", wie er hierin verwendet wird, bezieht sich auf einen Promotor, der im Ursprungsorganismus die Expression des betreffenden Gens kontrolliert.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promotor des Bhunenkohlmosaikvirus für pflanzliche Zellen, der Promotor der Alkoholdehydrogenase für Hefezellen, die T3-, T7- oder SP6-Promotoren für prokaryotische Zellen oderzellfreie Systeme.

Als Vektoren, die eine der genannten Nukleinsäuren, eine regulatorische Region oder ein DNA-Konstrukt enthalten, können alle in molekularbiologischen Laboratorien verwendete Phagen, Plasmide, Phagmide, Phasmide, Cosmide, YACs, BACs, künstliche Chromosomen oder Partikel, die für einen Partikelbeschuss geeignet sind, verwendet werden.

Bevorzugte Vektoren sind pBIN (Bevan, 1984) und seine Derivate für pflanzliche Zellen, pFL61 (Minet et al., 1992) oder z.B. die p4XXprom. Vektorserie (Mumberg et al.) für Hefezellen, pSPORT-Vektoren (Fa. Life Technologies) für bakterielle Zellen, lambdaZAP (Fa. Stratagene) für Phagen oder den Gateway Vektoren (Fa. Life Technologies) für verschiedene Expressionssysteme in bakteriellen Zellen oder in Baculovirus.

Im Rahmen der vorliegenden Erfindung werden auch Wirtszellen beschrieben, die eine der vorstehend genannten Nukleinsäuren, DNA-Konstrukte oder erfindungsgemäße Vektoren enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli*, als auch eukaryotische Zellen, wie Zellen von *Saccharomyces cerevisiae, Pichia pastoris*, Insekten, Pflanzen, Froschoozyten und Zelllinien von Säugern.

Im Rahmen der vorliegenden Erfindung wirden weiterhin Polypeptide mit der biologischen Aktivität von Phosphomevalonat Kinasen beschrieben die von den vorstehend genannten Nukleinsäuren codiert werden.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Diese Polypeptide können in der Form "reifer" Proteine oder als Teile größerer Proteine, z.B. als Fusionsproteine, vorliegen. Weiterhin können sie Sezernierungsoder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Diese Polypeptide, insbesondere das Polypeptid gemäß SEQ ID NO:2 müssen nicht vollständige pflanzliche Phosphomevalonat Kinasen darstellen, sondern können auch nur Fragmente davon sein, solange sie zumindest noch die biologische Aktivität der vollständigen pflanzlichen Phosphomevalonat Kinase aufweisen. Polypeptide, die eine gleichartige biologische Aktivität wie eine Phosphomevalonat Kinase mit einer Aminosäuresequenz gemäß SEQ ID NO:2 ausüben, werden noch als relevant betrachtet. Dabei müssen diese Polypeptide nicht von Phosphomevalonat Kinasen aus Arabidopsis ableitbar sein. Als verwendbar werden auch Polypeptide betrachtet, die Phosphomevalonat Kinasen beispielsweise der folgenden Pflanzen entsprechen oder Fragmenten davon, die noch die biologische Aktivität dieser ausüben können: Tabak, Mais, Weizen, Gerste, Hafer, Reis, Roggen, Tomaten, Leguminosen, Kartoffelpflanzen, *Lactuca sativa*, andere Brassicaceen, Holzgewächse, *Physcomitrolla patens*.

Die Polypeptide können im Vergleich zu der entsprechenden Region von natürlich vorkommenden Phosphomevalonat Kinasen Deletionen oder Aminosäuresubstitutionen aufweisen, solange sie zumindest noch die biologische Aktivität der vollständigen Kinase ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Im Rahmen der vorliegenden Erfindung werden somit auch Polypeptide als relevant betrachtet, welche zumindest die biochemische Reaktion der Bildung von 5-Pyrophosphomevalonat aus 5-Phosphomevalonat wie die Phosphomevalonat Kinase ausüben und eine Aminosäuresequenz umfassen, die eine zumindest 60 %ige Identität, vorzugsweise 80 %ige Identität, besonders bevorzugt 90 %ige Identität, ganz besonders bevorzugt 97-99 %ige Identität, mit der Sequenz gemäß SEQ ID NO: 2 über deren Gesamtlänge aufweist.

Der Grad der Identität der Aminosäuresequenzen wird vorzugsweise bestimmt mit Hilfe des Programms BLASTP + BEAUTY Version 2.0.14. (Altschul et al., 1997).

Eine besondere Ausführungsform der erfindungsgemäßen Polypeptide ist die Phosphomevelonat Kinase (PMVK) mit der Aminosäuresequenz gemäß SEQ ID NO: 2.

Die PMVK-Aminosäuresequenz besitzt im Bereich der Aminosäuren 177 bis 186 eine für Kinasen typische potentielle ATP- Bindungsstelle.

Der Ausdruck "biologische Aktivität einer Phosphomevalonat Kinase", wie er hierin verwendet wird, bedeutet die Fähigkeit zur Umsetzung von 5-Phosphomevalonat zu 5-Pyrophosphomevalonat unter Verbrauch von ATP und der Entstehung von ADP.

Die vorstehend genannten Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können auch verwendet werden, um ausgehend von Pflanzen-mRNA hergestellte cDNA-Banken zu durchsuchen. Klone, an die die markierten Oliogonukleotide hybridisieren, werden zur Isolierung der betreffenden DNA-Fragmente ausgewählt. Nach der Charakterisierung der isolierten DNA erhält man auf einfache Weise die beschriebenen Nukleinsäuren.

Die Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bezeichnet DNA-Moleküle, die aus 10 bis 50 Nukleotiden, vorzugsweise 15 bis 30 Nukleotiden, bestehen. Sie werden chemisch synthetisiert und können als Sonden verwendet werden.

Im Rahmen der Erfindung sind auch Polypeptide mit Phosphomevalonat Kinase Aktivität von Interesse, die von einer vorstehend genannten DNA kodiert werden.

Dem Fachmann ist bekannt, dass die genannten Polypeptide auf verschiedenem Wege gewonnen werden können, z.B. durch chemische Methoden wie der Festphasenmethode. Zur Gewinnung größerer Proteinmengen empfiehlt sich die Verwendung rekombinanter Methoden. Die Expression eines klonierten Phosphomevalonat Kinase Gens oder Fragmente davon kann in einer Reihe von passenden Wirtszellen erfolgen, die dem Fachmann bekannt sind. Zu diesem Zweck wird ein Phosphomevalonat Kinase Gen mit Hilfe bekannter Methoden in eine Wirtszelle eingeführt.

Zur Integration des klonierten Phosphomevalonat Kinase Gens in das Chromosom der Wirtszelle wird vorzugsweise das Gen oder Fragmente davon in ein Plasmid gebracht, und die kodierenden Regionen des Phosphomevalonat Kinase Gens oder Fragmente davon mit einem konstitutiven oder induzierbaren Promotor funktionell verknüpft.

Die grundlegenden Schritte zur Herstellung der rekombinanten Phosphomevalonat Kinase sind:
1. Gewinnung einer natürlichen, synthetischen oder semi-synthetischen DNA, die für die Phosphomevalonat Kinase kodiert.
2. Einbringen dieser DNA in einen Expressionsvektor, der geeignet ist, die Phosphomevalonat Kinase zu exprimieren, entweder alleine oder als Fusionsprotein.
3. Transformation einer passenden, vorzugsweise prokaryontischen Wirtszelle mit diesem Expressionsvektor.
4. Anzucht dieser transformierten Wirtszelle in einer Weise, die geeignet ist, die Phosphomevalonat Kinase zu exprimieren.
5. Ernte der Zellen und Aufreinigung der Phosphomevalonat Kinase durch geeignete, bekannte Methoden.

Die kodierende Region der Phosphomevalonat Kinase kann dabei mit den üblichen Methoden in *E. coli* exprimiert werden. Geeignete Expressionssysteme für *E*. *coli* sind kommerziell erhältlich, so die Expressionsvektoren der pET-Serie, z.B. pET3a, pET23a, pET28a mit His-Tag oder pET32a mit His-Tag zur einfachen Aufreinigung und Thioredoxinfusion zur Erhöhung der Löslichkeit des exprimierten Enzyms, sowie pGEX mit Glutathionsynthetase-Fusion, sowie die pSPORT Vektoren. Die Expressionsvektoren werden in λ DE3-lysogene *E. coli*-Stämme, z.B. BL21(DE3), HMS 174(DE3) oder AD494(DE3) transformiert. Nach dem Anwachsen der Zellen unter dem Fachmann geläufigen Standardbedingungen wird die Expression mit IPTG induziert. Nach Induktion der Zellen wird für 3 bis 24 Stunden bei Temperaturen von 18 bis 37°C inkubiert. Die Zellen werden durch Sonifikation in Aufschlusspuffer (10 bis 200 mM Natriumphosphat, 100 bis 500 mM NaCl, pH 5 bis 8, aufgeschlossen. Das exprimierte Protein kann über chromatographische Methoden gereinigt werden, im Fall von mit His-Tag exprimiertem Protein durch Chromatographie an einer Ni-NTA-Säule.

Die Expression des Proteins in kommerziell erhältlichen Hefestämmen (z.B. *Pichia pastoris*) oder in Insektenzellkulturen (z.B. Sf9-Zellen) stellt einen anderen günstigen Ansatz dar.

Alternativ können die Proteine auch in Pflanzen exprimiert werden.

Gegenstand der vorliegenden Erfindung sind Verfahren zum Auffinden von chemischen Verbindungen, die an die vorstehend genannten Polypeptide binden und deren Eigenschaften verändern. Aufgrund der vielfältigen Funktionen der Terpenoide, die die Bildung ihres Vorläufers Isopentyl-Diphosphat und damit die Funktion der vorstehend beschriebenen Phosphomevalonat Kinase notwendig machen, stellen Modulatoren, die die Aktivität des Enzyms beeinflussen, neue wuchsregulierende oder herbizide Wirkstoffe dar. Modulatoren können Agonisten oder Antagonisten bzw. Aktivatoren oder Inhibitoren sein.

Im Rahmen der vorliegenden Erfindung wird auch die Verwendung von pflanzlichen Phosphomevalonat Kinasen als Angriffspunkte für Herbizide und ihre Verwendung in Verfahren zum Auffinden von Modulatoren dieses Polypeptids beschrieben. In solchen Verfahren können die Phosphomevalonat Kinasen direkt, in Extrakten oder aufgereinigt eingesetzt werden, oder mittelbar über die Expression der dafür kodierenden DNA entstehen.

Die Verwendung von für pflanzliche PMVK kodierenden Nukleinsäuren, diese enthaltende DNA-Konstrukte, diese enthaltende Wirtszellen, oder von an PMVK bindenden Antikörpern zum Auffinden von Modulatoren der PMVK werden in Rahmen der vorliegenden Erfindung beschrieben.

Der Ausdruck "Agonist", wie er hierin verwendet wird, bezieht sich auf ein Molekül, das die Aktivität der Phosphomevalonat Kinase beschleunigt oder verstärkt.

Der Ausdruck "Antagonist", wie er hierin verwendet wird, bezieht sich auf ein Molekül, das die Aktivität der Phosphomevalonat Kinase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die vorstehend beschriebenen Polypeptide binden. Weiterhin können Modulatoren kleine organisch-chemische Moleküle, Peptide oder Antikörper sein, die an ein Molekül binden, welches wiederum an diese Polypeptide bindet, und dadurch deren biologische Aktivität beeinflusst. Modulatoren können natürliche Substrate und Liganden darstellen oder strukturelle oder funktionelle Mimetika davon. Der Ausdruck "Modulator" umfasst jedoch nicht die natürlichen Substrate sowie ATP.

Vorzugsweise handelt es sich bei den Modulatoren um kleine organisch-chemische Verbindungen.

Die Bindung der Modulatoren an die vorstehend beschriebenen Phosphomevalonat Kinasen kann die zellulären Vorgänge auf eine Weise verändern, die zum Absterben der damit behandelten Pflanzen führt.

Die erfindungsgemäßen Verfahren schließen Hochdurchsatz-Screening (high throughput screening; HTS) ein. Dafür können sowohl Wirtszellen als auch zellfreie Präparationen verwendet werden, die die vorstehend beschriebenen Nukleinsäuren und/oder die vorstehend beschriebenen Polypeptide enthalten.

Um Modulatoren der vorstehend genannten Polypeptide aufzufinden, kann ein synthetischer Reaktionsmix (z.B. Produkte der *in vitro*-Transkription) oder ein zellulärer Bestandteil, wie ein Zellrohextrakt, oder irgendeine andere Präparation, die dieses Polypeptid enthält, zusammen mit einem markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität eines der beschriebenen Polypeptide zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der Polypeptide führen, sind Agonisten. Moleküle, die gut binden, aber nicht die biologische Aktivität der Polypeptide auslösen, sind wahrscheinlich gute Antagonisten.

Die Detektion der biologischen Aktivität der Polypeptide kann durch ein sog. Reportersystem verbessert werden. Reportersysteme in dieser Hinsicht umfassen, sind aber nicht beschränkt auf colorimetrisch markierte Substrate, die in ein Produkt umgewandelt werden oder ein Reportergen, das auf Veränderungen der Aktivität oder der Expression der Polypeptide anspricht oder andere bekannte Bindungstests.

Modulatoren der vorstehend beschriebenen Polypeptide können auch über enzymatische Tests aufgefunden werden. Es kann entweder die Änderung der Enzymaktivität durch entsprechende Modulatoren direkt oder in einem gekoppelten Enzymtest indirekt gemessen werden. Die Messung kann z.B. über Absorptionsänderung durch die Ab- oder Zunahme einer optisch aktiven Verbindung durchgeführt werden.

Ein weiteres Beispiel für ein Verfahren, mit welchem Modulatoren der vorstehend beschriebenen Polypeptide aufgefunden werden können, ist ein Verdrängungstest, bei dem man unter dafür geeigneten Bedingungen die Polypeptide und einen potenziellen Modulator mit einem Molekül, das bekanntermaßen an diese Polypeptide bindet, wie einem natürlichen Substrat oder Liganden oder einem Substrat- oder Liganden-Mimetikum zusammenbringt. Die Polypeptide selbst können markiert werden, z.B. radioaktiv oder colorimetrisch, so dass man die Anzahl der Polypeptide, die an einen Liganden gebunden sind oder die eine Umsetzung mitgemacht haben, exakt bestimmen kann. Auf diese Weise lässt sich die Effektivität eines Agonisten oder Antagonisten ermessen.

### Beispiel 1

### Isolierung der für PMVK aus A. thaliana kodierenden Nukleinsäure

Mit Hilfe der Methode der "Supression subtractive hybridization" (Diatchenko et al., 1996) wurde mehrfach ein 370 bp Fragment der PMVK aus Blattmaterial von *Arabidopsis thaliana* cv. Columbia Pflanzen isoliert.

Die "Supression subtractive hybridization" stellt eine Methode zur Isolierung differentiell exprimierter Gene dar. Die zwei zu vergleichenden Proben waren zum einen Arabidopsispflanzen, die 24 Stunden nach Behandlung mit einem Herbizid (Chlorsulfuron, 10g/ha) geerntet wurden, und zum anderen Arabidopsispflanzen, die 24 Stunden nach einer Kontrollbehandlung geerntet wurden. Das 370 bp PMVK-Fragment wurde aus den mit Chlorsulfuron behandelten Pflanzen isoliert, in denen die Transkription der PMVK durch die Behandlung möglicherweise induziert worden war.

Das erhaltene Fragment wurde in den Vektor pTAdv (Firma Clontech) kloniert und in den E. coli Stamm TOP10F' transformiert. Das Fragment der PMVK wurde weiterhin als Sonde für virtual Northern (Firma Clontech) Blots verwendet und zur Isolierung der vollständigen cDNA von PMVK als Sonde eingesetzt.

### Isolierung der vollständigen cDNA Sequenz von PMVK

Es wurde eine Arabidopsis cDNA-Bibliothek der Firma Life Technologies im Plasmid-Vektor pSPORT mit Hilfe des Cloncapture Kits der Firma Clontech nach Angaben des Herstellers durchsucht. Im Unterschied zu den Angaben des Herstellers wurde jedoch die Markierung des als Sonde eingesetzten PMVK Fragments mit Biotin nicht mittels PCR sondern mit Hilfe des Biotin High Prime Kit der Firma Boehringer durchgeführt.

Die mit PMVK angereicherte Plasmid-DNA wurde in *E. coli* Zellen transformiert und über Nacht ausplattiert. Die erhaltenen Kolonien wurden durch Kolonie-PCR mit PMVK-genspezifischen Primern analysiert und positive Kolonien identifiziert.

Von den positiven Kolonien wurden mit dem Fachmann bekannten Methoden Kulturen angezogen, die Plasmid-DNA isoliert und die DNA anschließend sequenziert.

### Beispiel 2

Zur Überprüfung einer differentiellen Expression der PMVK in Reaktion auf Chlorsulfuron wurden so genannte virtual Northern Blot Analysen durchgeführt.

Bei einem virtual Northern Blot wird mit der SMART Methode der Firma Clontech (siehe Angaben des Herstellers) aus Gesamt-RNA cDNA hergestellt und mit PCR amplifiziert. Es werden nur so wenige PCR Zyklen eingesetzt, dass die Amplifikation sich noch im linearen Bereich der PCR befindet. In vorliegenden Fall zeigte sich ein Optimum zwischen 15 und 18 Zyklen. Die SMART cDNA wird nach dem Fachmann bekannten Methoden auf einem Agarose-Gel aufgetrennt, auf eine Nylonmembran übertragen und mit einer mit DIG markierten Sonde hybridisiert. Diese Methode erlaubt die Untersuchung der Expression auch von nur gering exprimierten Genen.

Das Ergebniss zeigte eine leichte Induktion der PMVK-Expression durch Chlorsulfuron.

### Beispiel 3

Ein mögliches Testsystem zur Identifizierung von Modulatoren der Phosphomevalonatkinase beruht auf dem ADP-Nachweis des gekoppelten Pyruvatkinase/Lactatdehydrogenase-Tests.

Phosphoenolpyruvat wird durch die Pyruvatkinase zu Pyruvat umgesetzt, dieses wird dann anschliessend von der Lactatdehydrogenase unter NADH-Verbrauch zu Lactat umgesetzt. Der NADH-Verbrauch lässt sich durch die Abnahme der Absorption bei 340 nm verfolgen.

Bei der Reaktion der PMVK wird ADP gebildet, welches im Rahmen der beschriebenen Tests nachgewiesen werden kann. Der Einfluss von Modulatoren der PMVK auf diese Reaktion kann damit auch anhand einer Erhöhung oder Erniedrigung des ADP Gehalts bestimmt werden.

### Abbildungen und Sequenzprotokoll

### Abbildung 1

Bestimmung der Homologie zwischen der erfindungsgemäßen Phosphomevalonat Kinase aus *A. thaliana* gemäß SEQ ID NO:2 und der bekannten Phosphomevalonat Kinase aus *S. cerevisiae* (BESTFIT) mittels Bestfit (Wisconsin Package Version 10.1 (GCG)). Die Ähnlichkeit beträgt 44 %, die Indentität 35 %.

### SEQ ID NO:1

Nukleinsäuresequenz kodierend für Phosphomevalonat Kinase aus *A. thaliana.*

### SEQ ID NO:2

Aminosäuresequenz der Phosphomevalonat Kinase aus *A. thaliana*.

### SEQ ID NO:3

Nukleinsäurefragment aus *Medicago trunculata* (putative PMVK) der Accession Nummer AA 660847.

### SEQ ID NO:4

Nukleinsäurefragment aus *Gossypium hirsutum* (putative PMVK) der Accession Nummer AI 727861.

### SEQ ID NO:5

Nukleinsäurefragment aus *Pinus radiata* (kodierend für PMVK gemäß WO 00/36081).

### Literatur

Altschul, S.F., Madden, T.L., Schaffer, A.A., Zhang, J.Z.; Miller W. and Lipman, D.J. 1997. Gapped BLAST und PSI-BLAST generation of protein database search programs. Nucleic Acids Res. 25: 3389-3402.

Bevan, M. 1984. Binary Agrobacterium vectors for plant transformation. Nucleic Acids Res 12(22): 8711-8721.

Croteau, R., 1992. Clomazone Does Not Inhibit the Conversion of Isopentyl Pyrophosphate to Geranyl, Farnesyl, or Geranylgeranyl Pyrophosphate *in Vitro.* Plant Physiol. 98, 1515-1517

Diatchenko, L., Lau, Y. C., Campbell, A. P., Chenchik, A., Moqadam, F., Huang, B., Lukyanov, S., Lukyanov, K., Gurskaya, N., Sverdlov, E. D., Siebert, P. D. 1996. Suppression subtractive hybridization: A method for generating differentially regulated or tissue-specific cDNA probes and libraries. Proc. Natl. Acad. Sci. USA 93, 6025-6030

Heintze, A., Görlach, J., Schulze-Siebert, D. Schultz, G. 1990. Plastidic isoprenoid synthesis during chloroplast development. Change from metabolic autonomy to division-of-labor stage. Plant Physiol. 93, 1121-1127

Lottspeich, F., Zorbas H. (Hrsg.). 1998. Bioanalytik. Spektrum Akademischer Verlag, Heidelberg, Berlin.

Minet, M., Dufour, M.-E. and Lacroute, F. 1992. Complementation of *Saccharomyces cerevisiae* auxotrophic mutants by *Arabidopsis thaliana* cDNAs. Plant J. 2: 417-422.

Mumberg, D., Müller, R., Funk, M.,.1995. Yeast vectors for the controlled expression of heterologous proteins in different genetic backgrounds. Gene 156, 119-122.

Norman, M. A., Liebl, R. A., Widholm, J. M., 1990. Site of Clomazone Action in Tolerant-Soybeyn and Susceptible-Cotton Photomixotrophic Cell Suspension Cultures. Plant Physiol. 94, 704-709

Weimar, M. R., Balke, N. E., Buhler, D. D., 1992. Herbicide Clomazone Does Not Inhibit *In Vitro* Geranylgeranyl Synthesis from Mevalonate. Plant Physiol. 98, 427-432

## Patentansprüche

1. Verfahren zum Identifizieren von herbiziden Wirkstoffen, indem man
(a) eine pflanzliche Phosphomevalonatkinase mit zumindest einer chemischen Verbindung in Kontakt bringt,
(b) die enzymatische Aktivität der Phosphomevalonatkinase bei Anwesenheit der chemischen Verbindung mit der Aktivität der Phosphomevalonatkinase bei Abwesenheit der chemischen Verbindung vergleicht, und
(c) die chemische Verbindung bestimmt, bei der die enzymatische Aktivität der Phosphomevalonatkinase gehemmt wird.

2. Verfahren gemäß Anspruch 1, **dadurch gekennzeichnet, dass** man
(a) das bei der Reaktion der Phosphomevalonatkinase entstehende ADP mit Hilfe einer Pyruvatkinase zu ATP umsetzt,
(b) das dabei entstehende Pyruvat mit Hilfe einer Lactatdehydrogenase unter NADH-Verbrauch zu Lactat umsetzt, und
(c) den Verbrauch des NADHs anhand einer Absorptionsänderung verfolgt.

3. Verfahren gemäß Anspruch 1 oder 2, **dadurch gekennzeichnet, dass** es sich bei der pflanzlichen Phosphomevalonatkinase um ein Polypeptid mit der Sequenz gemäß SEQ ID NO:2 oder ein Polypeptid mit einer zumindest 70%igen Identität mit der Sequenz gemäß SEQ ID NO:2 handelt.

## Claims

1. Method for identifying herbicidally active compounds by
(a) bringing a plant phosphomevalonate kinase in contact with at least one chemical compound,
(b) comparing the enzymatic activity of the phosphomevalonate kinase in the presence of the chemical compound with the activity of the phosphomevalonate kinase in the absence of the chemical compound, and
(c) identifying the chemical compound where the enzymatic activity of the phosphomevalonate kinase is inhibited.

2. Method according to Claim 1, **characterized in that**
(a) the ADP generated in the phosphomevalonate kinase reaction is converted into ATP with the aid of a pyruvate kinase,
(b) the pyruvate generated is converted into lactate with the aid of a lactate dehydrogenase with the consumption of NADH, and
(c) the NADH consumption is monitored with reference to a change in absorption.

3. Method according to Claim 1 or 2, **characterized in that** the plant phosphomevalonate kinase is a polypeptide with the sequence as shown in SEQ ID NO: 2 or a polypeptide with at least 70% identity with the sequence as shown in SEQ ID NO: 2.

## Revendications

1. Procédé d'identification de substances actives herbicides, dans lequel on
(a) met en contact une phosphomévalonate kinase végétale avec au moins un composé chimique,
(b) compare l'activité enzymatique de la phosphomévalonate kinase en présence du composé chimique avec l'activité de la phosphomévalonate kinase en l'absence du composé chimique, et on
(c) détermine le composé chimique avec lequel l'activité enzymatique de la phosphomévalonate kinase est inhibée.

2. Procédé selon la revendication 1, **caractérisé en ce que** l'on
(a) transforme l'ADP formé lors de la réaction de la phosphomévalonate kinase en ATP, à l'aide d'une pyruvate-kinase,
(b) transforme le pyruvate ainsi formé en lactate, à l'aide d'une lactate-déhydrogénase en consommant du NADH, et on
(c) suit la consommation de NADH à l'aide d'une modification de l'absorption.

3. Procédé selon la revendication 1 ou 2, **caractérisé en ce que** la phosphomévalonate kinase végétale est un polypeptide présentant la séquence selon SEQ ID NO:2 ou un polypeptide possédant une identité d'au moins 70 % avec la séquence selon SEQ ID NO:2.


### SEQUENZPROTOKOLL

<110> Bayer AG
<120> Phosphomevalonat Kinasen aus Pflanzen
<130> Le A 35 018
<140>
<141>
<160> 5
<170> PatentIn Ver. 2.1
<210> 1
<211> 2396
<212> DNA
<213> Arabidopsis thaliana
<220>
<221> CDS
<222> (685) .. (2199)
<400> 1
   gtcgacccac gcgtccgggc cgaccttctt cttcttcctt aagacaacac ataatgatag 60
   aagcaaactg gggaagatga agatggagtg gtgaagaaca aaaccgtata accgttcggt 120
   tcagaggtgc cgaaccgaac cgacccgtaa accgaaatcc tcaaaagaaa ttgccgatcg 180
   gtttgctact gttcaaaacc tcggtgccga gaaccgaaac tgtcggtttt ttcggttcgg 240
   gtttctcggt ttcttccgaa ctcccaggcc tagtttggtt ttatttttca cgagttttgc 300
   ttctcttttc atcggcgacg acgacgtcga gtttctgtca aaacgttaac gatccgactc 360
   gagcgtcgac agtaagagaa gaagacagcg attgtgtgta gatcgacggc gaacgtgtgt 420
   cgatccgtct cgatcgacgg agaatacgtt tcgatccggt ttcgatccaa atcggagagt 480
   ttgaggatct aaatcggaaa ttgcattaat actcatctcc aatctcttct gaagagtccg 540
   aatccgatct accaccacta ctcgtaccgc cggtcattta ctgccgccga tttcaaatta 600
   tccgatcatt tccggcgata tccaatcgca gactgaggtg aatctggggt tttgatcagc 660
   gattatcttt gtcactcttt gaaa atg gct gtt gtt gct tct gct cct ggg   711
                              Met Ala Val Val Ala Ser Ala Pro Gly
                              1               5
   aaa gtt ttg atg act gga ggc tac ctt gta ctc gag aag cca aat gca   759
   Lys Val Leu Met Thr Gly Gly Tyr Leu Val Leu Glu Lys Pro Asn Ala
   10                  15                  20                  25
   ggg ctt gtg ttg agt aca aat gca cgg ttt tac gcg att gtg aag cca   807
   Gly Leu Val Leu Ser Thr Asn Ala Arg Phe Tyr Ala Ile Val Lys Pro
                   30                  35                  40
   atc aac gaa gaa gtc aag cct gaa agt tgg gca tgg aaa tgg aca gat   855
   Ile Asn Glu Glu Val Lys Pro Glu Ser Trp Ala Trp Lys Trp Thr Asp
               45                  50                  55
   gtc aaa tta aca tca cca cag ctc tcg aga gaa agc atg tat aaa ctg    903
   Val Lys Leu Thr Ser Pro Gln Leu Ser Arg Glu Ser Met Tyr Lys Leu
           60                  65                  70
   tca ctg aat cat ttg act ctt cag tct gtg tct gca agt gat tca aga    951
   Ser Leu Asn His Leu Thr Leu Gln Ser Val Ser Ala Ser Asp Ser Arg
       75                  80                  85
   aac ccc ttt gta gag cat gcg ata cag tat gct ata gct gct gct cat    999
   Asn Pro Phe Val Glu His Ala Ile Gln Tyr Ala Ile Ala Ala Ala His
   90                  95                  100                 105
   ttg gca acc gag aag gac aaa gaa tca ttg cac aaa ctc tta ttg caa    1047
   Leu Ala Thr Glu Lys Asp Lys Glu Ser Leu His Lys Leu Leu Leu Gln
                   110                 115                 120
   ggt ctt gat ata aca ata tta ggc tcc aat gac ttt tac tca tat cgg    1095
   Gly Leu Asp Ile Thr Ile Leu Gly Ser Asn Asp Phe Tyr Ser Tyr Arg
               125                 130                 135
   aac cag ata gaa tcg gct ggg ctt cca ttg aca cca gaa tcg ctg ggt    1143
   Asn Gln Ile Glu Ser Ala Gly Leu Pro Leu Thr Pro Glu Ser Leu Gly
           140                 145                 150
   acc ctt gca ccg ttt gca tca atc aca ttc aat gct gcg gag tca aat    1191
   Thr Leu Ala Pro Phe Ala Ser Ile Thr Phe Asn Ala Ala Glu Ser Asn
       155                 160                 165
   ggt gct aat tcc aag cct gaa gta gca aaa act ggc tta ggt tct tct    1239
   Gly Ala Asn Ser Lys Pro Glu Val Ala Lys Thr Gly Leu Gly Ser Ser
   170                 175                 180                 185
   gca gca atg aca aca gct gtg gtt gca gct ctg tta cat tat ctt gga    1287
   Ala Ala Met Thr Thr Ala Val Val Ala Ala Leu Leu His Tyr Leu Gly
                   190                 195                 200
   gtg gtt gac cta tct gat cca tgt aaa gaa gga aag ttt ggc tgt tct    1335
   Val Val Asp Leu Ser Asp Pro Cys Lys Glu Gly Lys Phe Gly Cys Ser
               205                 210                 215
   gat cta gat gtt atc cat atg ata gca caa acg tct cat tgt ctt gca    1383
   Asp Leu Asp Val Ile His Met Ile Ala Gln Thr Ser His Cys Leu Ala
           220                 225                 230
   caa ggg aag gtc gga agt ggg ttt gat gtc agc tgt gct gtc tat gga    1431
   Gln Gly Lys Val Gly Ser Gly Phe Asp Val Ser Cys Ala Val Tyr Gly
       235                 240                 245
   agt cag cgt tat gtt cgc ttc tct cca gaa gtc ttg tca ttt gct cag    1479
   Ser Gln Arg Tyr Val Arg Phe Ser Pro Glu Val Leu Ser Phe Ala Gln
   250                 255                 260                 265
   gtt gca gta aca ggt ctg cca tta aat gaa gtt att ggt aca att ttg    1527
   Val Ala Val Thr Gly Leu Pro Leu Asn Glu Val Ile Gly Thr Ile Leu
                   270                 275                 280
   aag gga aaa tgg gac aat aag aga act gag ttc tct tta cca cca ctg    1575
   Lys Gly Lys Trp Asp Asn Lys Arg Thr Glu Phe Ser Leu Pro Pro Leu
               285                 290                 295
   atg aat ctt ttc ctt gga gaa cct gga agt ggt gga tcc tcc aca cca    1623
   Met Asn Leu Phe Leu Gly Glu Pro Gly Ser Gly Gly Ser Ser Thr Pro
           300                 305                 310
   tca atg gta ggt gca gta aag aag tgg caa atg tct gat cca gag aag    1671
   Ser Met Val Gly Ala Val Lys Lys Trp Gln Met Ser Asp Pro Glu Lys
       315                 320                 325
   gca cga gaa aac tgg cag aat ttg tca gat gca aat tta gaa ctg gaa    1719
   Ala Arg Glu Asn Trp Gln Asn Leu Ser Asp Ala Asn Leu Glu Leu Glu
   330                 335                 340                 345
   act aag cta aac gat ctg agc aaa tta gct aaa gac cac tgg gat gtt    1767
   Thr Lys Leu Asn Asp Leu Ser Lys Leu Ala Lys Asp His Trp Asp Val
                   350                 355                 360
   tat cta cga gtc att aag tct tgt agt gtg ctt act tct gaa aag tgg    1815
   Tyr Leu Arg Val Ile Lys Ser Cys Ser Val Leu Thr Ser Glu Lys Trp
               365                 370                 375
   gtg tta cat gct act gaa cca atc aac gaa gcc att att aaa gaa ctc    1863
   Val Leu His Ala Thr Glu Pro Ile Asn Glu Ala Ile Ile Lys Glu Leu
           380                 385                 390
   tta gag gca aga gaa gct atg ttg agg atc aga att ctt atg cgt cag    1911
   Leu Glu Ala Arg Glu Ala Met Leu Arg Ile Arg Ile Leu Met Arg Gln
       395                 400                 405
   atg ggt gag gcg gct agc gtt ccg ata gag cct gaa tct caa act caa    1959
   Met Gly Glu Ala Ala Ser Val Pro Ile Glu Pro Glu Ser Gln Thr Gln
   410                 415                 420                 425
   ctt ttg gat tct aca atg agt gct gaa gga gtt cta ctt gct ggt gtt    2007
   Leu Leu Asp Ser Thr Met Ser Ala Glu Gly Val Leu Leu Ala Gly Val
                   430                 435                 440
   cct gga gct ggt gga ttt gat gcc ata ttt gca atc act tta ggg gat    2055
   Pro Gly Ala Gly Gly Phe Asp Ala Ile Phe Ala Ile Thr Leu Gly Asp
               445                 450                 455
   tcc ggc acc aaa ctg acc cag gca tgg agt tcg cac aat gtt ttg gcc    2103
   Ser Gly Thr Lys Leu Thr Gln Ala Trp Ser Ser His Asn Val Leu Ala
           460                 465                 470
   ttg ttg gtg aga gaa gat cca cat ggc gtt tgc cta gaa agt ggt gat    2151
   Leu Leu Val Arg Glu Asp Pro His Gly Val Cys Leu Glu Ser Gly Asp
       475                 480                 485
   cca cga acc aca tgt att act tca ggc gtt tca tca att cac ctt gag    2199
   Pro Arg Thr Thr Cys Ile Thr Ser Gly Val Ser Ser Ile His Leu Glu
   490                 495                 500                 505
   taaacaacat tgtttcagtg tccaattatt aggtgcgtca ccaagttcgg ttgagtatac 2259
   tgttttgcat atagacttgg gtgctaaatt tcttggtgta agcattttta tacccattgt 2319
   aaggtcttta actcttggaa aacttgcggg aaaataaaat aaagttgatt tcaaatcttc 2379
   tcaaaaaaaa aaaaaaa                                                2396
<210> 2
<211> 505
<212> PRT
<213> Arabidopsis thaliana
<400> 2
   Met Ala Val Val Ala Ser Ala Pro Gly Lys Val Leu Met Thr Gly Gly
    1               5                  10                  15
   Tyr Leu Val Leu Glu Lys Pro Asn Ala Gly Leu Val Leu Ser Thr Asn
               20                  25                  30
   Ala Arg Phe Tyr Ala Ile Val Lys Pro Ile Asn Glu Glu Val Lys Pro
           35                  40                  45
   Glu Ser Trp Ala Trp Lys Trp Thr Asp Val Lys Leu Thr Ser Pro Gln
       50                  55                  60
   Leu Ser Arg Glu Ser Met Tyr Lys Leu Ser Leu Asn His Leu Thr Leu
   65                  70                  75                  80
   Gln Ser Val Ser Ala Ser Asp Ser Arg Asn Pro Phe Val Glu His Ala
                   85                  90                  95
   Ile Gln Tyr Ala Ile Ala Ala Ala His Leu Ala Thr Glu Lys Asp Lys
               100                 105                 110
   Glu Ser Leu His Lys Leu Leu Leu Gln Gly Leu Asp Ile Thr Ile Leu
           115                 120                 125
   Gly Ser Asn Asp Phe Tyr Ser Tyr Arg Asn Gln Ile Glu Ser Ala Gly
       130                 135                 140
   Leu Pro Leu Thr Pro Glu Ser Leu Gly Thr Leu Ala Pro Phe Ala Ser
   145                 150                 155                 160
   Ile Thr Phe Asn Ala Ala Glu Ser Asn Gly Ala Asn Ser Lys Pro Glu
                   165                 170                 175
   Val Ala Lys Thr Gly Leu Gly Ser Ser Ala Ala Met Thr Thr Ala Val
               180                 185                 190
   Val Ala Ala Leu Leu His Tyr Leu Gly Val Val Asp Leu Ser Asp Pro
           195                 200                 205
   Cys Lys Glu Gly Lys Phe Gly Cys Ser Asp Leu Asp Val Ile His Met
       210                 215                 220
   Ile Ala Gln Thr Ser His Cys Leu Ala Gln Gly Lys Val Gly Ser Gly
   225                 230                 235                 240
   Phe Asp Val Ser Cys Ala Val Tyr Gly Ser Gln Arg Tyr Val Arg Phe
                   245                 250                 255
   Ser Pro Glu Val Leu Ser Phe Ala Gln Val Ala Val Thr Gly Leu Pro
               260                 265                 270
   Leu Asn Glu Val Ile Gly Thr Ile Leu Lys Gly Lys Trp Asp Asn Lys
           275                 280                 285
   Arg Thr Glu Phe Ser Leu Pro Pro Leu Met Asn Leu Phe Leu Gly Glu
       290                 295                 300
   Pro Gly Ser Gly Gly Ser Ser Thr Pro Ser Met Val Gly Ala Val Lys
   305                 310                 315                 320
   Lys Trp Gln Met Ser Asp Pro Glu Lys Ala Arg Glu Asn Trp Gln Asn
                   325                 330                 335
   Leu Ser Asp Ala Asn Leu Glu Leu Glu Thr Lys Leu Asn Asp Leu Ser
               340                 345                 350
   Lys Leu Ala Lys Asp His Trp Asp Val Tyr Leu Arg Val Ile Lys Ser
           355                 360                 365
   Cys Ser Val Leu Thr Ser Glu Lys Trp Val Leu His Ala Thr Glu Pro
       370                 375                 380
   Ile Asn Glu Ala Ile Ile Lys Glu Leu Leu Glu Ala Arg Glu Ala Met
   385                 390                 395                 400
   Leu Arg Ile Arg Ile Leu Met Arg Gln Met Gly Glu Ala Ala Ser Val
                   405                 410                 415
   Pro Ile Glu Pro Glu Ser Gln Thr Gln Leu Leu Asp Ser Thr Met Ser
               420                 425                 430
   Ala Glu Gly Val Leu Leu Ala Gly Val Pro Gly Ala Gly Gly Phe Asp
           435                 440                 445
   Ala Ile Phe Ala Ile Thr Leu Gly Asp Ser Gly Thr Lys Leu Thr Gln
       450                 455                 460
   Ala Trp Ser Ser His Asn Val Leu Ala Leu Leu Val Arg Glu Asp Pro
   465                 470                 475                 480
   His Gly Val Cys Leu Glu Ser Gly Asp Pro Arg Thr Thr Cys Ile Thr
                   485                 490                 495
   Ser Gly Val Ser Ser Ile His Leu Glu
               500                 505
<210> 3
<211> 611
<212> DNA
<213> Medicago truncatula
<400> 3
   ctgttatctg agttgaagaa atatcacaat atcaatggcc gtggtggttg cttctgctcc 60
   tgggaaggtg ttaatgaccg gtggctacct agttttagag agacctaatg ctggacttgt 120
   tcttagtact aatgctcgtt tttatgctat tgtcaaacca atctatcctc aaactaaacc 180
   tgattcttgg gcttgggctt ggtcagatgt cagattaaca tctcctcaac tctccagaga 240
   agccttctat aaattagcac tcaaaaatct taccatccaa actgtttcct caagtgaaac 300
   aaggaaccct tttgtggaat atgctgtgca atactccgtg gctgccgcct atgcaacagc 360
   tgaccagaat aaaaaggact tgttgcacaa actacttttg caaggtcttg acattacaat 420
   tttgggttcc aatgattttt attcttatag gaatgagatt gagagacacg gactcccttt 480
   gacatcagaa tcattggcca cccttccgcc ttttgcctcc atttctttca atactgatga 540
   tgctaatgga aggaattgta agcctgaaat tgccaaaact ggtttgggct catctgcagc 600
   aatgacaacc g                                                      611
<210> 4
<211> 728
<212> DNA
<213> Gossypiurn hirsutum
<400> 4
   cgtttttacg ctattgttaa gccaattcat gaagctatca agcctgaaag ctgggcatgg 60
   tcttggaccg atgtcaagct aacatctcct cagctttcca gagaaagcat gtataaattg 120
   tctcggaaac atttaacact tcagtgtgta tcttcaagtg aatcaaggaa cccttttgta 180
   gaaaatgcta ttcaatatac tatagcagct gcacatgcaa catttgacaa gaataagaaa 240
   gaggcattag ataaactact cttacaaggt cttgatatta cgatcttagg ttgcaatgac 300
   ttttactcat acaggaatca gatagaagca cttggtcttc cgttgacacc tgaagcattg 360
   gctactctac caccgtttac atcaattaca ttcaattctg aggaatcaaa tggagcaaat 420
   tgcaaacctg aagttgcaaa aactggattg ggttcatctg cagcaatgac aactgctgta 480
   gttgctgctt tacttcatta tcttggtgtt gttaaccttt ccacctcttc tgcagatcaa 540
   caccaagaaa ataagaattc cacagatctc gatattgtgc atatgatagc tcaaagtgcc 600
   cactgtattg cccaaggtaa agttggcagt ggctttgatg tcagttctgc tgtctatggg 660
   agtcagcgtt atgttcgttt ttcaccaaaa gtgctttctg ctgctcaggc tgcantgaaa 720
   gggatgcc                                                         728
<210> 5
<211> 571
<212> DNA
<213> Pinus radiata
<400> 5
   cacaggcgaa accctctcct gctgctcacg gttgataaac cctcaatatt tgcggtaggg 60
   ctccagattt actgcaatct gccagtaaga gtccgttgtg gcggaagaga gctgccgaga 120
   gctgccgagc tggagagcac cattcgcacc atatagagaa gggggttgat agattcctgg 180
   tcaaggaaaa ctgacaataa ggtgaaaaaa acaataatta ccttcagatt atctgatcat 240
   cacatggctg tagttgtgtc agctcctggt aaggttttaa taacaggagc ttatctaatt 300
   cttgagaagc caaatccagg acttgtgctt accaccacag ctcgcttcta cgccattgtg 360
   aagccactgc ggactagcac agattccagt agttgggcat ggctatggac agatgtgaaa 420
   ttaacatcgc ctcagcttgc aaaggaggcc atctacaagc tatctctgaa gactcttagc 480
   ctgcaaaatg ttgcttcttc aagtagcaat ggtaatcctt ttgtggaaca agcagtgcaa 540
   tttgctgttg cagctgcaaa agaagccttt g                                 571